# EUROPEAN PATENT SPECIFICATION

(11) **EP 4 159 872 B1**
(45) Date of publication and mention of the grant of the patent: **11.09.2024**
(21) Application number: 22197543.6
(22) Date of filing: 24.06.2019
(51) Int. Cl.: C12Q 1/6841

(54) **LABELING OF DNA**
DNA-MARKIERUNG
MARQUAGE DE L'ADN

(30) Priority: 25.06.2018 US 201862689650 P; 11.07.2018 US 201862696696 P
(43) Date of publication of application: 05.04.2023
(62) Divisional of application: 19739487.7
(73) Proprietor: Bionano Genomics, Inc., San Diego, CA 92121 (US)
(72) Inventor: HASTIE, Alex, San Diego (US); ZHANG, Deng, San Diego (US)
(74) Representative: Hutter, Anton

(56) References cited:
- WO-A1-2015/075056
- WO-A1-2016/061523
- WO-A2-2017/040813
- WULAN DENG ET AL: "CASFISH: CRISPR/Cas9-mediated in situ labeling of genomic loci in fixed cells", PROCEEDINGS OF THE NATIONAL ACADEMY OF SCIENCES, vol. 112, no. 38, 31 August 2015 (2015-08-31), pages 11870 - 11875, XP055553074, ISSN: 0027-8424, DOI: 10.1073/pnas.1515692112

## Description

### RELATED APPLICATIONS

This application claims the benefit under 35 U.S.C. §119(e) of U.S. Provisional Patent Application Ser. No. 62/689,650, filed June 25, 2018, and U.S. Provisional Patent Application Ser. No. 62/696,696, filed July 11, 2018.

### REFERENCE TO SEQUENCE LISTING

The present application is being filed along with a Sequence Listing in electronic format. The Sequence Listing is provided as a file entitled Sequence_Listing_BNGEN_049WO.txt, created June 23, 2019, which is 1 kilobytes in size.

### FIELD

Embodiments herein relate generally to labeling DNA molecules, for example genomic labeling for analysis of linearized DNA in nanochannels.

### BACKGROUND

Genome mapping in fluidic nanochannels is a robust technology able to interrogate genome structural variation (SV) in megabase length DNA molecules outside the detection range of next generation sequencing (NGS). These genome mapping in fluidic channel technologies, such as nick label repair stain chemistry (NLRS) or directly labeled (non-damaging) using the direct label and stain chemistry (DLS) (both from Bionano Genomics, San Diego, CA) are able to generate structurally accurate genome assemblies for large and complex plant and animal genomes, including the 30 Gbp Axolotl genome.

The type II clustered regularly interspaced short palindromic repeats (CRISPR)-CRISPR-associated caspase 9 (Cas9) system derived from *Streptococcus pyogenes* has become a revolutionary tool for targeted genome editing because of its target sequence customizability and high binding and enzymatic efficiency. To achieve site-specific DNA recognition and cleavage, the protein Cas9 forms a riobonucleotprotein (RNP) complex with a duplex of CRISPR RNA (crRNA) and trans-activating crRNA (tracrRNA), which is partially complementary to the crRNA. The HNH and RuvC-like nuclease domains of Cas9 cut both DNA strands, generating double-stranded breaks (DSBs) at sites defined by an approximately 20-nucleotide seed sequence within an associated crRNA transcript. WO 2017/040813 A2 discloses a composition comprising a labeled nuclease-dead Cas9 (dCas) protein and a single guide ribonucleic acid sequence that is complementary to at least a portion of a deoxyribonucleic acid repeat expansion sequence. Deng et al. ("CASFISH: CRISPR/Cas9-mediated in situ labeling of genomic loci in fixed cells", 2015, PNAS 112(38): 11870-11875) discloses CRISPR/Cas9-mediated in situ labelling of genomic loci in fixed cells. WO 2016/061523 A1 discloses a probe comprising a Cas polypeptide, a gRNA specific for a target nucleic acid sequence that is complexed with the Cas polypeptide, and one or more labels bound to the Cas polypeptide, the gRNA, or both. WO 2015/075056 A1 discloses a method for selective in vitro isolation of double-stranded DNA.

### SUMMARY

The invention is as defined by the claims, and any other aspects, configurations, or embodiments set forth herein not falling within the scope of the claims are for information only. Some embodiments include a method of labeling a DNA at a target sequence. The method can comprise contacting the DNA with a first dCas protein, and a first labeled guide RNA (gRNA) comprising a first crRNA and a first tracrRNA comprising a first label, in which the first crRNA is complementary to a first target sequence of the DNA or a portion thereof. The method can comprise incubating the DNA, the first dCas protein, and the first labeled gRNA, in which the first dCas protein, the DNA, and the first labeled gRNA form a complex in which the first crRNA is hybridized to the first target sequence or portion thereof. Thus, the DNA can be labeled at a first target sequence to form a labeled DNA. In some embodiments, the method further comprises contacting the DNA with a second dCas protein, and a second labeled gRNA comprising a second crRNA and a second tracrRNA comprising a second label that is different from the first label, in which the second crRNA can be complementary to a second target sequence of the DNA or a portion thereof. The method can further comprise incubating the DNA with the second labeled gRNA, wherein the second dCAS protein, the DNA, and the second labeled gRNA form a second complex, in which the second crRNA is hybridized to the second target sequence or portion thereof. Thus, the DNA can be labeled at the second target sequence with the different label. In some embodiments, the DNA is contacted with the first labeled gRNA and the second labeled gRNA at the same time, and the DNA is incubated with the first dCas protein, the second dCas protein, the first labeled gRNA, and the second labeled gRNA at the same time. In some embodiments, the DNA remains intact throughout the method, including the contacting and the incubating steps, and until at least a labeled DNA is formed. In some embodiments, at least one of the first target sequence and the second target sequence is in a region comprising multiple repeats or structural variants not amenable enzymatic motif-based labeling such as nickase labeling. In some embodiments, at least one of the first target sequence and the second target sequence is in a region predicted to form and/or susceptible of forming a fragile site upon nick translation labeling. In some embodiments, the method further comprises selecting the first target sequence and/or the second target sequence as in a region comprising multiple repeats or structural variants not amenable to enzymatic motif-based labeling such as nickase labeling. In some embodiments, at least one of the first target sequence and the second target sequence is selected as in a region predicted to form and/or susceptible of forming a fragile site upon nick translation labeling. In some embodiments, at least one of the first target sequence and the second target sequence is comprised by a genomic region that would not comprise unevenly distributed labels upon nick labeling. In some embodiments, at least one of the first target sequence and the second target sequence is comprised by a repeated sequence. In some embodiments, the method does not comprise nick labeling. In some embodiments, the labeled DNA is capable of remaining intact upon linearization in a fluidic nanochannel. In some embodiments, the labeled DNA remains intact upon linearization in a fluidic nanochannel. In some embodiments, the method further comprises linearizing the labeled DNA in a fluidic nanochannel, in which the DNA remains intact upon said linearizing. In some embodiments, the method further comprises detecting a relative distance between the first label and the second label on the linearized DNA in the fluidic nanochannel. The fluidic nanochannel can, for example, have a length of at least 10 nm and a cross-sectional diameter of less than 1000 nm. In some embodiments, the firrst dCas is dCas9. In some embodiments, the second dCas is dCas9. At least one of the first dCas and the second dCas can comprise one or more mutations or one or more deletions in a HNH domain and/or RuvC-like domain. In some embodiments, the first dCas is not labeled. In some embodiments, the second dCas is not labeled. In some embodiments, at least one of the first crRNA and the second crRNA comprises a sequence of about 10-40 nucleotides that is complementary to one or more of the first and second target sequence or a portion thereof, for example about 10-30 nucleotides, about 10-20 nucleotides, about 15-30 nucleotides, about 15-25 nucleotides about 20-30 nucleotides, or about 20-40 nucleotides. In some embodiments, the incubating comprises one or more of the first dCas protein, the second dCas protein, the first labeled gRNA, and the second labeled gRNA at a concentration of about 120 nM per 150 ng of the DNA for hybridization (about 0.8 nM per ng DNA), for example no more than, or no more than about, 5 nM, 10 nM, 15 nM, 20 nM, 50 nM, 80 nM, 100 nM, 110 nM, 120 nM, 130 nM, 140 nM, 150 nM, 200 nM, 250 nM, 300 nM, 400 nM, 450 nM, or 500 nM per 150 ng of DNA, including ranges between any two of the listed values, for example, 5 nM to 25 nM, 5 nM to 20 nM, 5 nM to 15 nM, 5 nM to 500 nM, 10 nM to 25 nM, 10 nM to 20 nM, 10 nM to 15 nM, 10 nM to 500 nM, 50 nM to 500 nM, 100 nM to 500 nM, 120 nM to 500 nM, 5 nM to 300 nM, 10 nM to 300 nM, 50 nM to 300 nM, 100 nM to 300 nM, 120 nM to 300 nM, 5 nM to 200 nM, 10 nM to 200 nM, 50 nM to 200 nM, 100 nM to 200 nM, 120 nM to 200 nM, 5 nM to 120 nM, 10 nM to 120 nM, 50 nM to 120 nM, or 100 nM to 120 nM. In some embodiments, the concentration is 10 nM to 20 nM, for example 15 nM. Without being limited by theory, it has been observed that if the concentrations of these substances are too high, there can be high background that can interfere with the detection of true positive labels. In some embodiments, the incubating comprises one or more of the dCas protein and labeled gRNA at a concentration of about 120 nM per 150 ng of DNA for hybridization. In some embodiments, the method further comprises labeling the DNA by an additional chemistry, for example direct enzymatic labeling using an enzyme such as DLE-1 and optionally further including a stain in addition to the enzymatic labeling (e.g., BIONANO GENOMICS "DLS" technology), or nicking followed by nick labeling and repair (e.g., BIONANO GENOMICS "NLRS" technology) to produce a DNA with two or more different specificity motifs with different labels (e.g., different colors). In some embodiments, the nick labeling comprises nick translation, for example incubating the nicked DNA with a polymerase and labeled nucleotides, in which the nucleotides are labeled with a nucleotide label that is the same as or different from the label, whereby the polymerase incorporates the labeled nucleotides into the DNA in a 5' -> 3' direction. In some embodiments, the method does not comprise labeling the DNA by nick labeling comprising nick translation. In some embodiments, the method further comprises labeling with direct labeling enzyme such as DLE-1 (e.g., using DLS chemistry). The first label can be, for example, a fluorophore, a quantum dot, a dendrimer, a nanowire, a bead, a hapten, a streptavidin, an avidin, a neutravidin, a biotin, and a reactive group a peptide, a protein, a magnetic bead, a radiolabel, a non-optical label, or a combination of two or more of the listed items. In some embodiments, the second label is selected from the group consisting of: a fluorophore, a quantum dot, a dendrimer, a nanowire, a bead, a hapten, a streptavidin, an avidin, a neutravidin, a biotin, and a reactive group a peptide, a protein, a magnetic bead, a radiolabel, or a non-optical label, or a combination of two or more of the listed items. In some embodiments, the DNA is further labeled with a nonspecific label, for example a backbone label such as a YOYO label (the nonspecific label may also be referred to herein as a "stain"). The nonspecific label can be added after the CRISPR-dCas labeling. In some embodiments, the labeling of the method is performed in a single step.

Some embodiments include a DNA composition. The DNA composition can comprise a DNA molecule, a first dCas protein (such as dCas9) and a first labeled guide RNA (gRNA). The first labeled gRNA can comprise a first crRNA and a first tracrRNA comprising a first label. The first dCas, the first labeled gRNA, and the DNA can be comprised by a first complex comprising the first crRNA hybridized to a first target sequence of the DNA molecule or a portion thereof. In some embodiments, the DNA composition further comprises a second dCas protein and a second labeled gRNA comprising a second cRNA and a second tracrRNA labeled with a second label that is different from the first label. The second dCas protein, the second labeled gRNA, and the DNA can be comprised by a second complex comprising the second crRNA hybridized to a second target sequence of the DNA. In some embodiments, the DNA composition is linearized in a fluidic nanochannel. In some embodiments, the DNA is intact in the fluidic nanochannel. In some embodiments, the fluidic nanochannel has a length of at least 10 nm and a cross-sectional diameter of less than 1000 nm. In the DNA composition of some embodiments, the first dCas is dCas9. In the DNA composition of some embodiments, the second dCas is dCas9. In the DNA composition of some embodiments, the first dCas comprises one or more mutations or one or more deletions in a HNH domain and/or RuvC-like domain. In the DNA composition of some embodiments, the second dCas comprises one or more mutations or one or more deletions in a HNH domain and/or RuvC-like domain. In the DNA composition of some embodiments, the first dCas is not labeled. In the DNA composition of some embodiments, the second dCas is not labeled. In the DNA composition of some embodiments, at least one of the first and the second crRNAs comprises, consists essentially of, or consists of a sequence of about 10-40 nucleotides that is complementary to the first and/or the second target sequence or a portion thereof. In the DNA composition of some embodiments, the DNA comprises labeled nucleotides incorporated into the DNA, in which the nucleotides are labeled with a nucleotide label that is the same as or different from the first label and/or the second label. In some embodiments, the DNA is not nick-labeled. In some embodiments, the first label is selected from the group consisting of: a fluorophore, a quantum dot, a dendrimer, a nanowire, a bead, a hapten, a streptavidin, an avidin, a neutravidin, a biotin, and a reactive group a peptide, a protein, a magnetic bead, a radiolabel, or a non-optical label, or a combination of two or more of the listed items. In some embodiments, the second label is selected from the group consisting of: a fluorophore, a quantum dot, a dendrimer, a nanowire, a bead, a hapten, a streptavidin, an avidin, a neutravidin, a biotin, and a reactive group a peptide, a protein, a magnetic bead, a radiolabel, or a non-optical label, or a combination of two or more of the listed items. In some embodiments, the DNA is further labeled with a nonspecific label, for example a backbone label such as a YOYO label. In some embodiments, the DNA has been labeled with a direct labeling enzyme such as DLE-1.

Some embodiments include a kit for performing any of the labeling methods described herein. The kit can comprise a dCas protein as described herein (such as dCas9). In some embodiments, the kit further comprises a label. In some embodiments, the label is not attached to the dCas protein. In some embodiments, the kit further comprises a gRNA comprising a crRNA and a tracrRNA. In some embodiments, the gRNA comprises the label, for example comprised by the tracrRNA. In some embodiments, the kit further comprises a nickase. In some embodiments, the kit further comprises a direct labeling enzyme such as DLE-1.

### BRIEF DESCRIPTION OF THE DRAWINGS

**FIGS. 1A-1E** are a series of schematic diagrams showing a method for labeling a DNA according to some embodiments herein.
**FIG. 2A** is a fluorescent micrograph of labeled DNA in nanochannels according to some embodiments herein.
**FIG. 2B** is a graph of aligned molecules by label pattern according to some embodiments herein.
**FIGS. 3A-3B** are each graphs depicting labeled human genomic DNA after nick labeling and binding of a labeled CRISPR-dCAS9 complex according to some embodiments herein. **FIG. 3A** shows the Genomic mapping on CHM1 by dCas9/Alu-Atto647. **FIG. 3B** depicts Alu repeat mapping on CHM1 by dCas9/Alu.
**FIGS. 4A-4B** are schematic diagrams which show fragmentation that can occur at fragile sites as a result of nicking, where nicks are closer to one another (**FIG. 4A**), as well as nicks that mover farther apart, inhibiting the risk of fragile site formation (**FIG. 4B**).
**FIGS. 5A-5D** are a series of schematic diagrams showing a method for labeling a DNA according to some embodiments herein.

### DETAILED DESCRIPTION

The invention is as defined by the claims, and any other aspects, configurations, or embodiments set forth herein not falling within the scope of the claims are for information only. According to the labeling methods, DNA compositions, and kits of some embodiments herein, DNA is labeled using modified nuclease-deficient clustered regularly interspaced short palindromic repeats (CRISPR)/CRISPR-associated caspase (Cas; for example CRISPR-associated Caspase 9, "Cas9"), complexes as probes, which label sequence-specific genomic loci fluorescently without DNA nicking. The Cas can be a nuclease-deficient Cas (dCas) protein such as dCas9. The DNA-CRISPR-dCas complex can be assembled *in vitro.* Advantageously, the dCas/gRNA complex (such as dCas9/gRNA) stably bind its target, and is amenable to simultaneous probing of multiple targets with differently colored dCas/gRNA complexes (such as dCas9/gRNA), allowing multicolor labeling of targets. For example a first gRNA can comprise a first label and a first crRNA that is complementary to a first target sequence on the DNA or a portion thereof. A second gRNA can comprise a second label (that is different from the first label) and a second crRNA that is complementary to a second target sequence (that is different from the first target sequence) or a portion thereof on the DNA. The first gRNA can form a first complex with dCas (such as dCas9) at the first target sequence of the DNA, and the second gRNA can form a second complex with dCas (such as dCas9) at the second target sequence of the DNA. In some embodiments, the DNA labeled with the first complex (comprising the first label) and second complex (comprising the second label) can be linearized in a fluidic nanochannel. The relative distance between the first label and the second label on the linearized DNA can be measured. Since the gRNAs comprise labels, the dCas (such as dCas9) can be unlabeled in some embodiments.

In the labeling methods, DNA compositions, and kits of some embodiments disclosed herein, DNA is directly labeled with a fluorescently-labeled CRISPR-dCas complex (such as a CRISPR-dCas9 complex) as described herein. The labeling methods can comprise labeling DNA comprising targeting labels to selected target sequences on the DNA using dCas (such as dCas9), which has no nuclease activity but maintains its targeting specificity and efficiency when directed by target RNAs. By labeling a tracrRNA with a lablel, such as Atto647, and producing a complex that targets a genomic region, labeling can be accomplished by direct binding. Using nuclease-deficient Cas9 (dCas9) protein assembled with fluorescently labeled tracrRNAs combined with various crRNAs, rapid labeling of repetitive Duff1220 elements and Alu loci is demonstrated herein *(See* **Examples 1** and **2**, respectively). In some embodiments, the labeling method, in addition to labeling with CRISPR-dCas, further comprises labeling the DNA by an additional chemistry, for example direct enzymatic labeling using an enzyme such as DLE-1 and optionally further including a stain in addition to the enzymatic labeling (e.g., BIONANO GENOMICS "DLS" technology,), or nicking followed by nick labeling and repair (e.g., BIONANO GENOMICS "NLRS" technology), to produce a DNA comprising two or more specificity motifs (such as target sequences) with different labels (e.g., different colors). It is contemplated that labeling multiple specificity motifs with multiple colors can yield greater information density than labeling a fewer number of motifs. Advantageously, the labeling methods herein can be accomplished with a simple protocol that only requires incubation, and it is non-damaging to DNA. It is further contemplated that the forces associated with linearizing and mobilizing DNA in fluidic nanochannels can cause double-stranded breakage of damaged DNA, confounding the analysis of labeling patterns. Accordingly, in some embodiments herein, the CRISPR-dCas (such as CRISPR-dCas9)-labeled DNA are undamaged (and thus also intact), which can be amenable to analysis of the labeled DNA via linearization in fluidic nanochannels as described herein. Without being limited by theory, it is contemplated the labeling methods herein can achieve labeling more rapidly, and be used to target a greater variety of target sequences than nick labeling. In some embodiments, the labeling method does not comprise nick labeling.

Methods of labeling DNAs of some embodiments described herein can be useful in, for example, targeting repetitive sequences, barcoding genomic regions and structural variants not amenable to enzymatic motif-based labeling, for example nicking followed by direct enzymatic labeling using an enzyme such as DLE-1 (e.g., BIONANO GENOMICS "DLS" technology), or nickase-based methods such as nicking followed by nick labeling and repair (e.g., BIONANO GENOMICS "NLRS" technology) that can involve uneven distributions of motifs in the DNA such as nickase motifs. This rapid, convenient, non-damaging and cost-effective technology provides a valuable tool for both automated high-throughput genome-wide mapping as well as targeted analyses of complex regions containing repetitive and structurally variant DNA. Accordingly, in some embodiments, a method of labeling DNA comprises selecting a target sequence in a genomic region that is not amenable to labeling with motif-based labeling such as nick translation that would require uneven distributions of restriction site motifs in the DNA. The method can comprise using a gRNA as described herein comprising a crRNA complementary to a target sequence in the genomic region or a portion thereof that is not amenable to labeling with nick translation. In some embodiments, the method of labeling DNA comprises selecting a target sequence in a genomic region that comprises multiple repeats (such as Alu or Duff1220), barcoding genomic regions, and/or structural variants not amenable to enzymatic-motif based labeling such as nickase-based labeling methods (for example nick translation). In some embodiments, the method of labeling DNA comprises contacting a DNA with a panel of labeled gRNAs specific for SNPs. In some embodiments, the method of labeling DNA comprises labeling a customized target. In some embodiments, the method of labeling DNA comprises labeling a barcode sequence.

In some embodiments, the labeling method, in addition to labeling with CRISPR-dCas, further comprises labeling the DNA by an additional chemistry, for example direct enzymatic labeling using an enzyme such as DLE-1 and optionally further including a stain in addition to the enzymatic labeling (e.g., BIONANO GENOMICS "DLS" technology,), or nicking followed by nick labeling and repair (e.g., BIONANO GENOMICS "NLRS" technology) to produce a DNA with two or more specificity motifs (such as target sequences) labeled with different labels (e.g., different colors). In some embodiments, the nick labeling comprises nicking the DNA with a modified restriction enzyme which cuts a single strand (nickase) instead of both strands. Labeled nucleotides can then be incorporated into the nicked DNA directly (optionally, followed by repair), or by nick translation. Optionally, the DNA can be repaired with ligase following the nick translation. Optionally, the DNA can also be stained with a non-specific backbone label, such as a YOYO label. The nonspecific label can be added after the CRISPR-dCas labeling. This nick labeling process can be extremely efficient and produce high-resolution whole genome assemblies. However, enzyme motif-based labeling processes such as direct enzymatic labeling (e.g., DLS) and nick labeling are limited by the number of target sequences in available enzymes. Accordingly, in some embodiments, nick labeling is performed in conjunction with CRISPR-dCas (such as dCas9) labeling as described herein, which can provide a greater variety of target sequences.

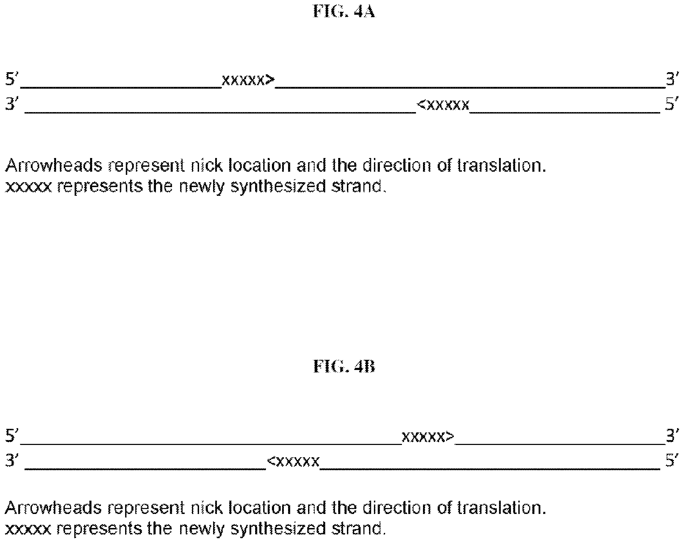

It is further contemplated that as part of a nick labeling process comprising nick translation, when there are nicks on opposite strands on the same DNA, the nicks can either move closer together (as shown in Figure 4A) or farther apart (as shown in Figure 4B). Without being limited by theory, it has been observed that fragile sites occur when two nicks are <1Kb apart on opposite DNA strands. Fragmentation can occur at fragile sites due to, for example, (1) mechanical manipulation, (2) heat required for labeling, (3) strand extension associated with labeling and certain kinds of repair (e.g., using the exonuclease activity of polymerases), and/or (4) shear forces associated with linearizing DNA molecules. Thus, labeling with CRISPR-dCas in accordance with some embodiments herein can be used to maintain strand integrity in regions predicted to give rise to fragile sites during nick translation.

### dCas proteins

As used herein, a "dCas protein" has its customary and ordinary meaning as understood by one of skill in the art in view of this disclosure. It refers to a class of Cas proteins (such as a class of Cas9) without nuclease activity. The dCas protein (such as dCas9) can lack the nuclease activity, but bind to a guide RNA (gRNA)-DNA complex with high specificity and efficiency. It is noted that there are multiple types of Cas proteins, including for example, Cas1 through Cas9. In accordance with labeling methods, DNA compositions, and kits of some embodiments, the dCas protein is a dCas9 protein. In the labeling methods, DNA compositions, and kits of some embodiments, the dCas protein (such as the dCas9 protein) does not nick the DNA. It is contemplated that nuclease activity can be inhibited or prevented in dCas proteins by one or more mutations and/or one or more deletions in the HNH and/or RuvC-like domains of the dCas protein. In accordance with labeling methods, DNA compositions, and kits of some embodiments, the dCas (such as dCas9) protein comprises one or more mutations in one or both of the HNH and RuvC-like domains. In some embodiments, the dCas (such as dCas9) protein comprises a mutation in the HNH domain, for example a point mutation, a frameshift, an insertion, a deletion of some or all of the domain, or an insertion-deletion deleting some or all of the domains. In some embodiments, the dCas (such as dCas9) protein comprises one or more mutations in the RuvC-like domain, for example a point mutation, a frameshift, an insertion, a deletion of some or all of the domain, or an insertion-deletion deleting some or all of the domains. In some embodiments, the dCas (such as dCas9) protein comprises one or more mutations in the HNH domain, for example a point mutation, a frameshift, an insertion, a deletion of some or all of the domains, or an insertion-deletion deleting some or all of the domain, and a mutation in the RuvC-like domain, for example a point mutation. In some embodiments, the dCas (such as dCas9) protein comprises a mutation in the HNH domain, for example a point mutation, a frameshift, an insertion, a deletion of some or all of the domain, or an insertion-deletion deleting some or all of the domain, and a mutation in the RuvC-like domain, for example a frameshift. In some embodiments, the dCas (such as dCas9) protein comprises a mutation in the HNH domain, for example a point mutation, a frameshift, an insertion, a deletion of some or all of the domain, or an insertion-deletion deleting some or all of the domain, and a mutation in the RuvC-like domain, for example an insertion. In some embodiments, the dCas (such as dCas9) protein comprises a mutation in the HNH domain, for example a point mutation, a frameshift, an insertion, a deletion of some or all of the domain, or an insertion-deletion deleting some or all of the domain, and a mutation in the RuvC-like domain, for example a deletion of some or all of the RuvC-like domain. In some embodiments, the dCas9 protein comprises a mutation in the HNH domain, for example a point mutation, a frameshift, an insertion, a deletion of some or all of the domain, or an insertion-deletion deleting some or all of the domain, and a mutation in the RuvC-like domain, for example an indention-deletion deleting some or all of the RuvC-like domain. In methods and DNA compositions of some embodiments, the dCas9 protein is unlabeled. It is noted that a Cas9 D10A mutant has one of its cleavage sites deactivated, which can convert the CRISPR-Cas9 complex into a nickase with customizable target sequences. While Cas9 D10A can be used for labeling similar to restriction-enzyme based nick labeling (for example, involving nicking, labeling, and repair by ligation, as separate steps), as Cas9 D10A still has nuclease (nicking) activity, it will be understood that Cas9 D10A does not represent a dCAS as used herein. As such, in some embodiments, the dCAS does not comprise Cas9D10A.

### gRNAs, crRNAs, and tracrRNAs

As used herein, a "guide RNA (gRNA)" has its customary and ordinary meaning as understood by one of skill in the art in view of this disclosure. It refers to a class of RNAs that comprises, consists essentially of, or consists of a trans-activating crRNA (tracrRNA) and a crRNA. The crRNA can comprise, consist essentially of, or consist of a sequence that is complementary to a target sequence on a DNA or a portion thereof as described herein. By way of example and without any limitations, the crRNA can comprises, consists essentially of, or consists of a sequence of, or a sequence of at least, 10, 15, 20, 25, 30, 35, or 40 nucleotides that are complementary to the target sequence or a portion thereof, including ranges between any two of the listed values, for example, 10-15 nucleotides, 10-20 nucleotides, 10-25 nucleotides, 10-30 nucleotides, 10-35 nucleotides, 10-40 nucleotides, 15-20 nucleotides, 15-25 nucleotides, 15-30 nucleotides, 15-35 nucleotides, 15-40 nucleotides, 20-25 nucleotides, 20-30 nucleotides, 20-35 nucleotides, 20-40 nucleotides, 25-30 nucleotides, 25-35 nucleotides, 25-40 nucleotide, 30-35 nucleotides, 30-40 nucleotides, or 35-40 nucleotides complementary to the target sequence or a portion thereof. The tracrRNA can comprise any of the labels described herein. The tracrRNA can be at least partially complementary to the crRNA. Without being limited by theory, binding of the tracrRNA to the crRNA at the target sequence of the DNA or portion thereof, can result in the formation of a complex with Cas9 at the target sequence. In some embodiments, the tracrRNA and the crRNA are part of a single molecule (in *cis*). In some embodiments, the the tracrRNA and the crRNA are parts of separate molecules. It is contemplated that in accordance with labeling methods, DNA compositions, and kits of some embodiments, the crRNA is complementary to a portion of the target sequence (e.g., is not complementary to the full length of the target sequence of interest). In some embodiments, the crRNA is complementary to a customized target sequence. In some embodiments, the crRNA is complementary to a barcode sequence.

It is contemplated that in the labeling methods, DNA compositions, and kits of some embodiments, two or more different labeled gRNAs can be used to label different target sequences of a DNA. Accordingly, it is contemplated that in the labeling methods, DNA compositions and kits of some embodiments, two or more gRNAs can each comprise a different label as described herein. Accordingly, in the labeling methods, DNA compositions and kits of some embodiments, the DNA labeling is multiplex.

### Fluidic nanochannels

Fluidic nanochannels can be useful for the analysis of structural features of long (e.g., kiliobase, or megabase-length) DNA molecules as well as short DNA molecules. Detailed information on suitable fluidic nanochannels can be found, for example, in U.S. Patent Nos. 8722327, 8628919, and 9533879. Suitable nanochannels for the labeling methods, DNA compositions, and kits of some embodiments, can have, for example, a diameter of less than about twice the radius of gyration of the macromolecule in its extended form. A nanochannel of such can exert entropic confinement of the freely extended, fluctuating DNA coils so as to extend and elongate the DNA.

Suitable fluidic nanochannel segments of the methods and DNA compositions and kits of some embodiments can for example have a characteristic cross-sectional dimension of less than about 1000nm; or less than about 500 nm; or less than about 200 nm, or less than about 100 nm; or less than about 50 nm, about 10 nm, about 5 nm, about 2 nm; or less than about 0.5 nm. A fluidic nanochannel segment, in some embodiments, has a characteristic cross-sectional dimension of less than about twice the radius of gyration of the DNA molecule. In some embodiments, the nanochannel has a characteristic cross-sectional dimension of at least about the persistence length of the DNA molecule. Fluidic nanochannel segments suitable for the methods, kits, and DNA compositions of some embodiments herein can have, for example, a length of at least about 100 nm, of at least about 500 nm, of at least about 1000 nm, of at least about 2 microns, of at least about 5 microns, of at least about 10 microns, of at least about 1 mm, or of at least about 10 mm. Fluidic nanochannel segments are, in some embodiments, present at a density of at least 1 fluidic nanochannel segment per cubic centimeter.

Accordingly, in the labeling methods, DNA compositions, and kits of some embodiments, the fluidic nanochannel is capable of linearizing the DNA molecule (so as to entropic confinement of the DNA coils so as to extend and elongate the DNA molecule). Upon linearization in a fluidic nanochannel, the DNA molecule is maintained in a linearized, stretched conformation that permits the determination of the relative positons of labels along the length of the DNA. Such labels can be used to study DNA structural variations such as complex rearrangements, haplotype analysis, quantification of copy number of repeater elements on long (kilobase or megabase-scale) DNA, quantify short DNAs, resolve multiple repeats, insertions, and/or to assemble sequences or labeling patterns indicative of DNA structures onto a scaffold.

### Methods of labeling a DNA molecule

In some embodiments, a method of labeling a DNA molecule at a target sequence is described (such methods may also be described herein as "labeling methods"). The labeling method can comprise contacting the DNA with a dCas protein (such as dCas9) as described herein. The labeling method can comprise contacting the DNA with a labeled gRNA as described herein. The labeled gRNA can comprise a crRNA complementary to a target sequence or a portion thereof as described herein, and a tracrRNA comprising a label. The dCas protein, DNA, and labeled gRNA form a complex wherein the crRNA is hybridized to the target sequence. Thus, the DNA can be labeled by the labeling method. In some embodiments, the DNA is contacted with the gRNA and the dCas protein (such as dCas9) at the same time. In some embodiments, the DNA is contacted with the gRNA and the dCas protein (such as dCas9) in a single composition. In some embodiments, the DNA is contacted with the gRNA and the dCas protein (such as dCas9) in separate compositions, either at the same time or at different times. In some embodiments, the labeling of the method is performed in a single step. In some embodiments, the labeled DNA has a length in the kilobase or megabase range, for example at least 1 kb, 2 kb, 3kb, 5 kb, 10 kb, 20 kb, 30 kb, 40 kb, 50 kb, 100 kb, 150 kb, 250 kb, 500 kb, 1 Mb, 1.5 Mb, or 2 Mb, including ranges between any two of the listed values (for example 1 kb-2 Mb, 5 kb-2 Mb, 10 kb-2 Mb, 20 kb-2 Mb, 100 kb-2 Mb, 500 kb-2Mb, 1 kb-1 Mb, 5 kb-1 Mb, 10 kb-1 Mb, 100 kb-1 Mb, 200 kb-1 Mb, 500 kb-1Mb, 1 kb-500 kb, 5 kb-500 kb, 10 kb-500 kb, 20 kb-500 kb, 100 kb-500 kb, 1 kb-100 kb, 5 kb-100 kb, 10 kb-100 kb, 20 kb-100 kb, 50 kb-100 kb, 1 kb-50 kb, 5 kb-50 kb, 10 kb-50 kb, 1 kb-10 kb, 5 kb-10 kb, or 1 kb-5 kb).

In some embodiments, the labeling method includes labeling the DNA at two or more different target sequences using different labels for each target sequence. Accordingly, the labeling method or complex of some embodiments, further comprises two or more gRNAs that each comprises a crRNA that is complementary to a different target sequences or portion(s) thereof of the DNA, and a tracRNA labeled with a different label, so that different target sequences on the DNA are labeled with different labels. In some embodiments, each target sequence is labeled with a unique label. For example, the labeling method can comprise contacting the DNA with a first gRNA comprising a crRNA complementary to a first target sequence (or portion thereof) on the DNA and a tracrRNAs comprising a first label, a second gRNA comprising a crRNA complementary to a second target sequence (or portion thereof) on the DNA that is different from the first target sequence and a tracrRNA comprising a second label that is different from the first label, and/or a third gRNA comprising a crRNA complementary to a third target sequence (or portion thereof) on the DNA that is different from the first target sequence and/or the second target sequence and a tracrRNA comprising a third label that is different from the first label and/or the second label.

In some embodiments, the labeling method further comprises contacting the DNA with at least a second labeled gRNA comprising a second crRNA and a second tracrRNA comprising a second, different label, in which the second crRNA is complementary to a second, different target sequence or a portion thereof. The labeling method can comprise incubating the DNA with the second labeled gRNA, in which dCAS protein (such as dCas9), the DNA, and the second labeled gRNA form a second complex, in which the second crRNA (and thus the second, different label) is hybridized to the second, different target sequence or portion thereof on the DNA. Thus, the DNA can be labeled at least two different target sequences with at least two different labels. In some embodiments, the DNA is contacted with the first labeled gRNA and the second labeled gRNA at the same time, for example in a single composition. As such the first labeled gRNA and the second labeled gRNA can be incubated with the DNA and the dCAS protein (such as dCAS9) at the same time. In some embodiments, the DNA is contacted with the first labeled gRNA and the second labeled gRNA separately. For example, the DNA can be contacted with a first composition comprising the first labeled gRNA and a second, separate composition comprising the second labeled gRNA. In some embodiments, the DNA is contacted with the first and second compositions at the same time, and the first labeled gRNA and the second labeled gRNA are incubated with the DNA and the dCAS protein (such as dCSA9) at the same time. In some embodiments, the DNA is contacted with the first and second compositions at different times, and the first labeled gRNA and the second labeled gRNA are incubated with the DNA and the dCAS protein (such as dCAS9) at different times. In some embodiments, the DNA is contacted with the first and second compositions at different times, and the first labeled gRNA and the second labeled gRNA are incubated with the DNA and the dCAS protein (such as dCAS9) for different, but overlapping time periods (for example, if the first and second compositions are added sequentially).

It is contemplated that the dCAS (such as dCAS9) does not create double stranded breaks in the DNA, and thus can be useful for maintaining integrity of DNA strands during and after the labeling. In the labeling method of some embodiments, the DNA remains intact (e.g., double stranded without breaks) throughout the method. In the labeling method of some embodiments, the DNA remains intact after linearization and/or transport through a fluidic nanochannel as described herein. In the DNA compositions of some embodiments, the DNA remains intact (e.g., double stranded without breaks) after being labeled. In the DNA compositions of some embodiments, the DNA remains intact while linearized in a fluidic nanochannel as described herein. In some embodiments, the target sequence of the DNA is selected so as to avoid breaks that could be caused by nick labeling (with or without nick translation). For example, in the labeling methods, DNA compositions, and kits of some embodiments, the target sequence is selected as being in a region that predicted to form, or is susceptible for forming, a fragile site upon nick translation labeling (*See*, *e.g.*, **FIG. 4A**). In the first labeling methods, DNA compositions, and kits of some embodiments, the target sequence is in a region that predicted to form a fragile site upon nick translation labeling. In the labeling methods, DNA compositions, and kits of some embodiments, the labeled DNA is capable of remaining intact upon linearization in a fluidic nanochannel. In the labeling methods, DNA compositions, and kits of some embodiments, the labeled DNA remains intact upon linearization in a fluidic nanochannel.

In accordance with the labeling methods of some embodiments, the target sequence is selected as being comprised by a genomic region that would not comprise unevenly distributed labels upon enzymatic motif-based labeling such as nick labeling, for example a genomic region comprising multiple repeated sequences. In the labeling methods, DNA compositions, and kits of some embodiments, the target sequence is comprised by a genomic region that would not comprise unevenly distributed labels upon enzymatic motif-based labeling such as nick labeling, for example a genomic region comprising multiple repeated sequences. In accordance with the labeling methods of some embodiments, the target sequence is selected as being comprised by one or more repeated sequences (for example, repeated elements such as Alu elements or Duff1220 elements). In the labeling methods, DNA compositions, and kits of some embodiments, the target sequence is comprised by one or more repeated sequences. Examples of repeated elements that can comprise the target sequence in accordance with the labeling methods, compositions, and kits of some embodiments include, but are not limited to, Alu elements and Duff1220 elements. In accordance with the labeling methods of some embodiments, the target sequence is selected as comprising a SNP. In some embodiments, the method comprises contacting the DNA with a panel of gdRNAs specific for SNPs. Each gDRNA of the panel can be specific for a different SNP and comprise a different label. In some embodiments, the labeling methods comprises labeling a customized target. In some embodiments, the labeling method comprises labeling a barcode sequence.

In the labeling methods of some embodiments, the method further comprises linearizing the labeled DNA in a fluidic nanochannel. The DNA can, for example, remain intact upon linearizing. By way of example and without any limitation, the fluidic nanochannel can have a length of at least 10 nm and a cross-sectional diameter of less than 1000 nm, for example, less than 900 nm, 800 nm, 700 nm, 600 nm, 500 nm, 400 nm, 300 nm, 200 nm, or 100 nm. In some embodiments, the method further comprises detecting a relative distance between two labels as described herein on the linearized DNA in the nanochannel. In some embodiments, the method comprises detecting a relative distance between two labeled CRISPR-dCas complexes as described herein on the linearized DNA in the nanochannel.

In the labeling methods of some embodiments, the dCas protein comprises, consists essentially of, or consists of a dCas9 protein as described herein. In labeling methods of some embodiments that involve forming two or more labeled CRISPR-dCas complexes, all of the dCas proteins can comprise, consist essentially of, or consist of dCas9 as described herein. In some embodiments, the dCas (such as dCas9) comprises a mutation or deletion in a HNH domain and/or RuvC-like domain as described herein. In the labeling method of some embodiments, the dCas protein (such as dCas9) is not labeled. In the labeling method of some embodiments that comprise multiple CRISPR-dCas complexes, none of the dCas proteins (such as dCas9) is labeled.

In the labeling method of some embodiments, the crRNA comprise a sequence of about 10-40 nucleotides that is complementary to the target sequence or portion thereof as described herein.

In the labeling method of some embodiments, the DNA, dCas protein, and labeled gRNA are incubated so as to label the DNA in a single incubation. In the labeling method of some embodiments, the DNA, dCas protein, and/or the labeled gRNA are labeled at a concentration of at least about1 ng/µl, for example at least about 1, 2, 3, 4, 5, 6, ,7, 8, 9, 10, 15, 20, 25, 30, 35, 40, 45, 50, 60, 70, 80, 90, 100, 110, 120, 130, 140, 150, 160, 170, 180, 190, 200, 250, 300, 350, 400, 450, or 500 ng/µl, including ranges between any two of the listed values, for example, about 1 ng/µl to about 10 ng/µl, about1 ng/µl to about 50 ng/µl, about 1 ng/µl to about 100 ng/µl, about 1 ng/µl to about 200 ng/µl, about 1 ng/µl to about 300 ng/µl, about 1 ng/µl to about 500 ng/µl, about 5 ng/µl to about 10 ng/µl, about 5 ng/µl to about 50 ng/µl, about 5 ng/µl to about 100 ng/µl, about 5 ng/µl to about 200 ng/µl, about 5 ng/µl to about 300 ng/µl, about 5 ng/µl to about 500 ng/µl, about 10 ng/µl to about 50 ng/µl, about 10 ng/µl to about 100 ng/µl, about 10 ng/µl to about 200 ng/µl, about 10 ng/µl to about 300 ng/µl, about 10 ng/µl to about 500 ng/µl, about 50 ng/µl to about 100 ng/µl, about 50 ng/µl to about 200 ng/µl, about 0 ng/µl to about 300 ng/µl, or about 50 ng/µl to about 500 ng/µl.

In some embodiments, the labeling method, in addition to labeling with CRISPR-dCas, further comprises labeling the DNA by an additional chemistry, for example direct enzymatic labeling using an enzyme such as DLE-1 and optionally further including a stain in addition to the enzymatic labeling (e.g., BIONANO GENOMICS "DLS" technology,), or nicking followed by nick labeling and repair (e.g., BIONANO GENOMICS "NLRS" technology) of DNA to produce a DNA comprising two or more specificity motifs (such as target sequences) with different labels (e.g., different colors). In some embodiments, the labeling method further comprises labeling the DNA by nick labeling comprising nick translation so as to incorporate labeled nucleotides as described herein. In some embodiments, the nick translation comprises nicking the DNA with a nickase, and incubating the nicked DNA with a polymerase and labeled nucleotides. The nucleotides can be labeled with a nucleotide label that is the same as or different from the label. The polymerase can incorporate the labeled nucleotides into the DNA in a 5' -> 3' direction.

As disclosed herein, non-limiting exemplary labels include: a fluorophore, a quantum dot, a dendrimer, a nanowire, a bead, a hapten, a streptavidin, an avidin, a neutravidin, a biotin, a reactive group a peptide, a protein, a magnetic bead, a radiolabel, a non-optical label, and a combination of two or more of the listed items. In some embodiments, the label is an optical label. If the labeling method comprises two or more different labels, then two or more of the labels can be of the same types (for example two different fluorophores), or two or more of the different labels can be of two or more different types (for example, a fluorophore and a quantum dot), or a combination of two or more of the listed items. In the labeling methods, DNA compositions, and kits of some embodiments, the DNA is further labeled with a nonspecific label, for example a backbone label such as a YOYO label.

### Kits

Some embodiment include a kit for performing any of the labeling methods as described herein. The kit can comprise a dCas protein as described herein, for example a dCas9 protein. In some embodiments, the kit comprises a label. In some embodiments, the label is not attached to the dCas protein In some embodiments, the kit comprises a gRNA. The gRNA can comprise a label, for example any label described herein. In some embodiments, the gRNA comprises a crRNA and a tracrRNA as described herein. The crRNA can be complementary to a specified DNA target sequence or a portion thereof, for example a repeat sequence such as Alu or Duff1220. In some embodiments, the crRNA is complementary to a SNP. In the kit of some embodiments, the kit further comprises a nickase as described herein, for example Nt.BspQI. In some embodiments, the nickase is modified to nick only one strand of a DNA. In some embodiments, the kit further comprises a fluidic nanochannel as described herein. In some embodiments, the kit further comprises a direct-labeling enzyme. The direct-labeling enzyme can be a non-nicking enzyme, such as DLE-1. In some embodiments, the kit further comprises DLE-1.

### DNA Compositions

Some embodiments include DNA compositions. The DNA compositions can comprise, consists essentially of, or consist of a DNA, a dCas protein (such as dCas9) as described herein, and a labeled gRNA comprising a crRNA and a tracrRNA as described herein. The dCas (such as dCas9), the labeled gRNA, and the DNA can be comprised by a complex comprising the crRNA hybridized to a target sequence of the DNA or a portion thereof.

In some embodiments, the DNA of the DNA composition is linearized in a fluidic nanochannel as described herein. As it is contemplated that the CRISPR-dCas labeling of some embodiments herein does not nick or cut the DNA, in some embodiments, the DNA of the DNA composition can be intact in the nanochannel (e.g., does not have any double-stranded breaks compared to the pre-labeled DNA). In some embodiments, the fluidic nanochannel has a length of at least 10 nm and a cross-sectional diameter of less than 1000 nm. In some embodiments, the DNA of the DNA composition has a length in the kilobase or megabase range, for example at least 1 kb, 2 kb, 3kb, 5 kb, 10 kb, 20 kb, 30 kb, 40 kb, 50 kb, 100 kb, 150 kb, 250kb, 500 kb, 1 Mb, 1.5 Mb, or 2Mb, including ranges between any two of the listed values (for example 1 kb-2 Mb, 5 kb-2 Mb, 10 kb-2 Mb, 20 kb-2 Mb, 100 kb-2 Mb, 500 kb-2 Mb, 1 kb-1 Mb, 5 kb-1 Mb, 10 kb-1 Mb, 100 kb-1 Mb, 200 kb-1 Mb, 500 kb-1 Mb, 1 kb-500 kb, 5 kb-500 kb, 10 kb-500 kb, 20 kb-500 kb, 100 kb-500 kb, 1 kb-100kb, 5 kb-100 kb, 10 kb-100 kb, 20 kb-100 kb, 50 kb-100 kb, 1 kb-50 kb, 5 kb-50 kb, 10 kb-50kb, 1 kb-10 kb, 5 kb-10 kb, or 1 kb-5 kb). In some embodiments, the DNA of the DNA composition maintains its integrity (and length) after linearization in a fluidic nanochannel as described herein.

In some embodiments, the dCas of the DNA composition comprises one or more mutations and/or one or more deletions in a HNH domain and/or RuvC-like domain as described herein. In some embodiments, the DNA composition comprises a dCas9 as described herein. In some embodiments, for DNA compositions that comprise two or more dCas-CRIPSR labels, each dCas is a dCas9 as described herein. In some embodiments, for DNA compositions that comprise two or more dCas-CRIPSR labels, each dCas comprises a mutation or deletion in a HNH domain and/or RuvC-like domain as described herein. In some embodiments, the dCas of the DNA composition is not itself labeled. In some embodiments, no dCas of the DNA composition is labeled.

In some embodiments, the gRNA of the DNA composition comprises a crRNA complementary to a target sequence or portion thereof on the DNA as described herein. The crRNA can comprise a sequence of about 10-40 nucleotides that is complementary to the target sequence or portion thereof, as described herein. It is contemplated that the target sequence can comprise, consist essentially of, or consist of any sequence amenable to CRISPR targeting (for example, by substituting the crRNA probe, a desired sequence can be targeted).

In some embodiments, the DNA of the DNA composition is further nick labeled as described herein. Accordingly, in some embodiments, the DNA of the DNA composition comprises labeled nucleotides. The nucleotides can be labeled with a nucleotide label that is the same as or different from the label on the gRNA that also labels the DNA. In the DNA compositions of some embodiments, each label (gRNA and/or nucleotide label) of the DNA is selected from the group consisting of: a fluorophore, a quantum dot, a dendrimer, a nanowire, a bead, a hapten, a streptavidin, an avidin, a neutravidin, a biotin, and a reactive group a peptide, a protein, a magnetic bead, a radiolabel, or a non-optical label, and a combination of two or more of the listed items. In the DNA compositions of some embodiments, the label comprises an optical label. In some embodiments, for example DNA compositions that comprise two or more labels, the DNA of the DNA composition comprises two or more labels that are different from each other, but are each of the same type (for example, two different colors of fluorophores). In some embodiments, for example DNA compositions that comprise two or more labels, the DNA of the DNA composition comprises two or more labels that are different from each other, and of different types (for example, a fluorophore and a quantum dot). In some embodiments, the DNA is further labeled with a nonspecific label, for example a backbone label such as a YOYO label.

### Additional Embodiments

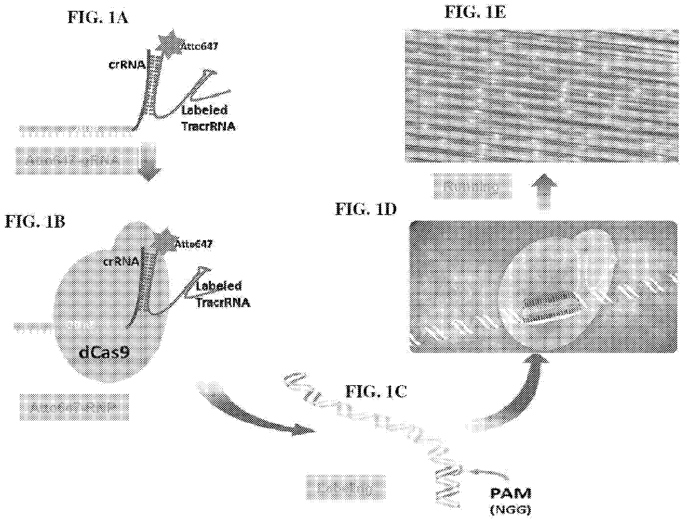

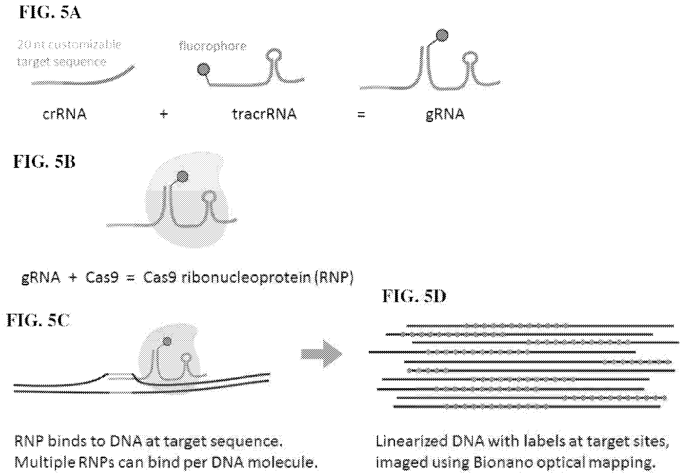

The dCas (such as dCas9) bind-labeling system of methods and DNA compositions of some embodiments herein uses a guide RNA (gRNA) to direct the dCas (such as dCas9) to a targeted site. The gRNA comprises, consists essentially of, or consists of a fluorescent-conjugated trans-activating crRNA (tracrRNA) and a crRNA that contains a ~20 nucleotide sequence that is complementary to the site of interest. Double mutations in the HNH and RuvC-like domain nuclease alters the Cas9 enzyme to bind without catalytic activity at very high specificity and efficiency. In some embodiments herein, complexes are labeled by synthesizing TracrRNA with a labeled (such as Atto647) instead of labeling of the dCas9 protein. With dCas9, labeled TracrRNA and a custom crRNA probe, any sequence amenable to CRISPR targeting can be targeted by substituting the crRNA probe. This DNA labeling method, CRISPR-bind, has been applied to labeling BAC DNA and megabase length human genomic DNA and imaging of this DNA in nanochannel arrays using the SAPHYR or IRYS system (Bionano Genomics, San Diego, CA, *See* **FIGS. 1A-1E** and 5A-5D).

Cas9 nuclease-deficient derivatives (dCas9) can also be used for control of gene expression and visualization of genomic loci in live cells through fusion with a transcription-regulation domain or a fluorescent protein, respectively. *In vitro* studies of the CRISPR system indicated that dCas9/ sgRNA had a strong and stable affinity for its target DNA.

Described in accordance with the methods, DNA compositions, and kits of some embodiments herein is sequence-specific labeling with dCas9 protein. The method is rapid, convenient cost-effective, and non-damaging. The flexible and efficient fluorescent tagging of specific sequences allows the ability to obtain context specific sequence information along the long linear DNA molecules in the SAPHYR nanochannel array (Bionano Genomics, San Diego, CA). Not only can this integrated fluorescent DNA double strand labeling make the whole genome mapping more accurate, and provide more information, but it can also specifically target certain loci for clinical testing, including detection of SNPs. Additionally, it can render the labeled double-stranded DNA available in long intact stretches for high-throughput analysis in nanochannel arrays as well as for lower throughput targeted analysis of labeled DNA regions using alternative methods for stretching and imaging the labeled large DNA molecules. Thus, labeling methods of some embodiments dramatically improve both automated high-throughput genome-wide mapping as well as targeted analyses of complex regions containing repetitive and structurally variant DNA. dCas9 fluorescent binding system, allowing for developing combinatorial, multiplexed, multicolor imaging systems, and thus can offer advantages for rapid genetic diagnosis of structural variations.

Sequence-specific labeling method with dCas protein in accordance with the methods and kits of some embodiments described herein can be useful in optical mapping. This single-step labeling of some embodiments does not damage the DNA, and the flexible and efficient fluorescent tagging of specific sequences enables acquisition of context-specific sequence information, when performing single-molecule optical mapping in nanochannel arrays such as the Bionano Genomics IRYS or SAPHYR system. Not only can the methods and kits of some embodiments yield superior quality and sensitivity of whole-genome structural variation analysis by adding a second color and increasing information density, it is also able to target a wide variety of sequences such as long tandem repeats, viral integration sites, transgenes, and can even be used to genotype single nucleotide variants.

### References

The following references are included for completeness of the disclosure:
McCaffrey, J., Sibert, J., Zhang, B., Zhang, Y., Hu, W., Riethman, H., and Xiao, M. (2016) CRISPR-CAS9 D10A nickase target-specific fluorescent labeling of double strand DNA for whole genome mapping and structural variation analysis. Nucleic Acids Res. Jan 29; 44(2): e11
Bittel DC, Z. X. (2014). Ultra high-resolution gene centric genomic structural analysis of a non-syndromic congenital heart defect, Tetralogy of Fallot. PLoS One, 9 (1), e87472.
Bochukova EG, H. N.-S.-S. (2010). Large, rare chromosomal deletions associated with severe early-onset obesity. Nature, 463 (7281), 666-70.
Brand H, P. V. (2014). Cryptic and complex chromosomal aberrations in early-onset neuropsychiatric disorders. Am J Hum Genet., 95 (4), 454-61.
Butcher NJ, K. T. (2013). Association between early-onset Parkinson disease and 22q11.2 deletion syndrome: identification of a novel genetic form of Parkinson disease and its clinical implications. JAMA Neurol., 70 (11), 1359-66.
Chaisson MJ, H. J. (2015). Resolving the complexity of the human genome using single-molecule sequencing. Nature, 517 (7536), 608-11.
Chen B, Gilbert LA, Cimini BA, et al. (2013) Dynamic Imaging of Genomic Loci in Living Human Cells by an Optimized CRISPR/Cas System. Cell. 2013; 155(7):1479-1491
Deininger PL, Batzer MA. (1999) Alu repeats and human disease. Mol Genet Metab. 1999 Jul;67(3): 183-93. Review.
Deng, W, Shi X, Tjian R, Lionnet T, Singer. (2015) CASFISH: CRISPR/Cas9-mediated in situ labeling of genomic loci in fixed cells. Proc. Natl. Acad. Sci., 112 (38), 11870-11875
Dominguez AA, Lim WA, Qi LS.(2016) Beyond editing: repurposing CRISPR-Cas9 for precision genome regulation and interrogation. Nat Rev Mol Cell Biol. 2016 Jan; 17(1):5-15
Dumas, LJ. et al. (2012) DUF1220-Domain Copy Number Implicated in Human Brain-Size Pathology and Evolution. American Journal of Human Genetics 91.3: 444-454
Gasiunas G, Barrangou R, Horvath P, Siksnys V. (2012) Cas9-crRNA ribonucleoprotein complex mediates specific DNA cleavage for adaptive immunity in bacteria. Proc. Natl. Acad. Sci. 109, E2579-E2586
Jinek M, Chylinski K, Fonfara I, Hauer M, Doudna JA, Charpentier E. (2012) A programmable dual-RNA-guided DNA endonuclease in adaptive bacterial immunity. Science 337, 816-821
Lo A and Qia L. (2017) Genetic and epigenetic control of gene expression by CRISPR-Cas systems.
Version 1. F1000Res. 2017; 6: F1000 Faculty Rev-747. doi: 10.12688/f1000research.11113.1
McCaffrey J, Young E, Lassahn K, Sibert J, Pastor S, Riethman H and Xiao M (2017) High-throughput single-molecule telomere characterization. Genome Res. 2017. 27: 1904-1915
Qi LS, Larson MH, Gilbert LA, Doudna JA, Weissman JS, Arkin AP, Lim WA. (2013) Repurposing CRISPR as an RNA-guided platform for sequence-specific control of gene expression. Cell. 2013 Feb 28; 152(5):1173-83.
Sternberg SH, Doudna JA (2015) Expanding the Biologist's Toolkit with CRISPR-Cas9. Mol Cell 58(4):568-574.

### EXAMPLES

Some aspects of the embodiments discussed above are disclosed in further detail in the following examples, which are not in any way intended to limit the scope of the present disclosure.

### Example 1: Labeling Duff1220 repeats on BAC DNA by CRISPR-bind

CRISPR-bind was used to target the Duff1220 repeat present on chromosome 1. The Duff1220 repeat sequence is present in variable copy number organized in several tandem arrays of the repeat unit, the copy number has been correlated with brain size in primates. Long tandem repeats can be challenging to measure since they are longer than sequence reads and too short to visualize by FISH or other cytogenetic methods. Optical mapping of ultra-long molecules that span the repeat arrays can allows the repeat units to be counted and the arrays placed into context; however, the repeats are typically not marked by conventional labeling enzymes.

A target probe was synthesized based on Mccaffery et al. and a CRISPR complex was produced with fluorescently labeled tracrRNA. The complex was incubated with BAC DNA anticipated to contain 13 copies of Duff1220 after linearization. CRISPR-bind complex concentration was titrated to determine a desirable concentration in this assay, which was about 15 nM. At this concentration, the signal-to-noise ratio (SNR) allowed the sample to be loaded directly onto the SAPHYR chip without having to clean up excess fluorophores, which is contemplated to simplify the method. 120 nM per 150 ng of DNA for hybridization (about 0.8 nM per ng DNA) was determined to be the desirable concentration (data not shown). From the images, the efficiency of detection of labels at each binding site was measured as well as the frequency that a signal was called outside of the expected Duff1220 loci. Target binding efficiency was 86% while about 16% of labels were found to be off-target. This performance is similar to standard nick labeling with Nt.BspQI.

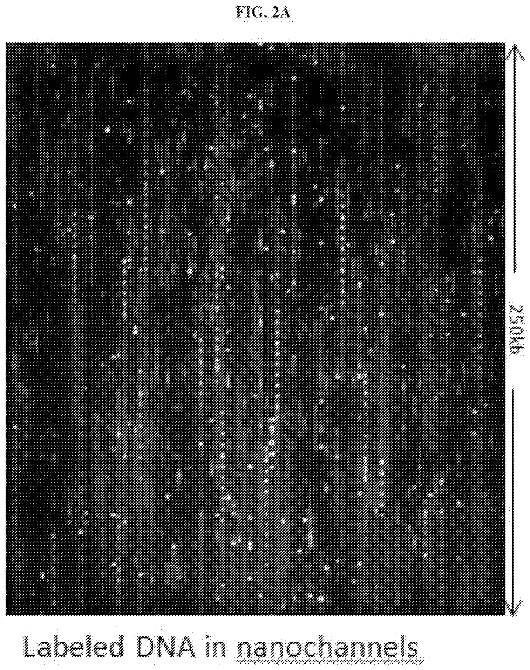

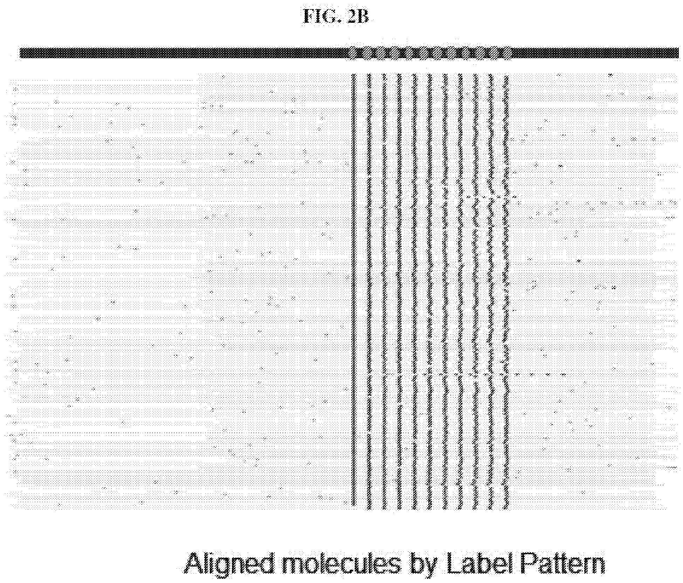

The results of the dCas9/Duf1220-Atto647 labeling on BAC of B19 are shown in FIGS. 2A-B. The expected labeling pattern and distances between labels is shown above corresponding fluorescent microscopy images. As shown in **FIG. 2A**, a single copy of DUF1220 triplet is 4.7 kb. The dCas9 labeling method was able to label this region using a gRNA designed to be complementary to the DUF1220 repeat unit. The tandem repeats are separated approximately 4.7 kb as predicted. **FIG. 2B** shows the histogram of the DUF1220 gRNA labels and clustered molecules. **Table 1** shows calculation for Alignment Rate for **FIG. 2B****.**

**Table 1. Alignment Rate**

| Samples | FP/(/100kb) | FPrate | FN(rate) | alignRate% |
|---|---|---|---|---|
| Atto647-gRNA | 1.0 | 16.1% | 13.7% | 88.1 |

Thus, long DNA with CRISPR-dCas labeling in accordance with some embodiments herein yields accurate labeling with similar accuracy to nick labeling.

### Example 2: Labeling human genomic DNA after nick labeling to define and localize Alu sequences

In order to test CRISPR-bind for specificity and efficiency on human genomic DNA, another target sequence which binds to about a quarter of Alu repeats across the genome was chosen. This target has the significant advantage that is occurs very frequently in the genome and is also interesting to study because it is mobile and is involved in structural variation and disease. Alu repeats are the most abundant transposable elements in the human genome, and they can copy themselves and insert into new locations, sometimes altering gene expression and even causing disease (Deininger 1999). Unlike some other potential CRISPR ~20-bp targets, this Alu repeat occurs very frequently in the genome allowing for relatively high density of labels across the genome. Utilization of CRISPR-bind-Alu can add 70% additional labels to a standard genome mapping experiment compared to nick-labeling alone or about 50% more compared to DLS using DLE-1 alone, potentially providing higher information density for genomic variation analysis. Also, since the CRISPR-bind can be in an alternate color, the second color signal can provide additional pattern uniqueness.

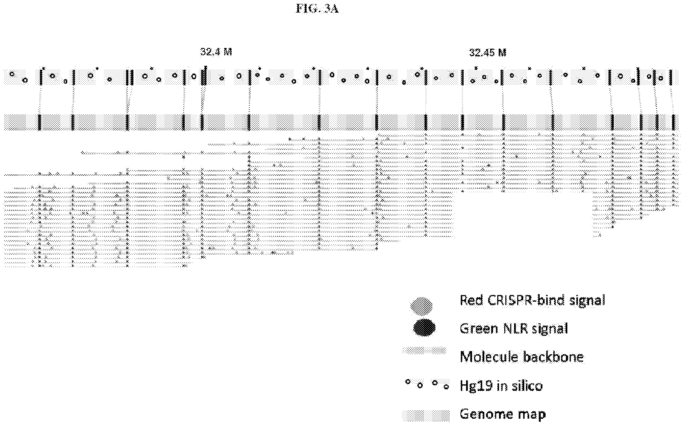

In order to estimate specificity and efficiency of binding and detection, Nt.BspQI sites were first labeled with standard nick translation based labeling using a green dye followed by binding our CRISPR-bind complex targeting Alu (red dye atto647) on a human pseudo-haploid hydatidiform mole cell line, CHM1. Data was collected and mapped based on the Nt.BspQI pattern to the human reference. **FIG. 3A** shows these molecules aligned, the Nt.BspQI signal is in dark purple and the Alu signal is shown in magenta. The magenta signal occurs in a consistent pattern in all of the molecules such that approximately 90% of all molecules mapping to a particular region contain signal the same signal near a mapped nick label. When location of the Alu repeats that have been targeted by the complex was annotated, they form a concordant pattern. While in some instances a consensus of molecules showed labels inconsistent to the reference loci, this is consistent with the high degree of variation of Alu patterns from individual to individual. **FIG. 3A** shows the Genomic mapping on CHM1 by dCas9/Alu-Atto647.

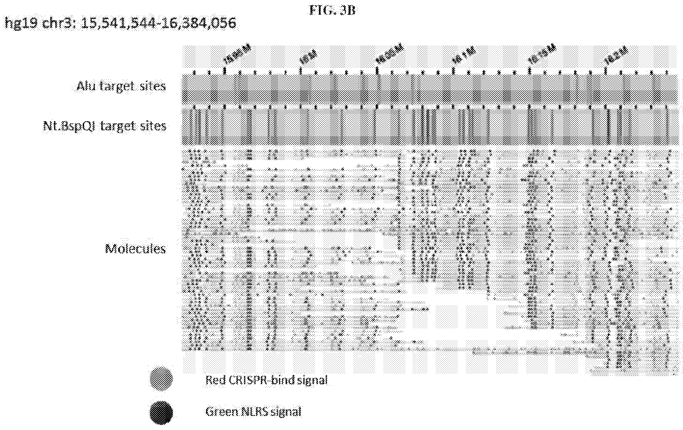

**FIG. 3B** depicts Alu repeat mapping on CHM1 by dCas9/Alu. Molecules labeled with both NLRS and +CRISPR-bind, labeled molecules aligned by their nick-label patterns to an in silico digestion of hg19 reference with Nt.BspQI. Predicted Alu target sites are shown above. Nt.BspQI signal is shown on the molecules in purple and Alu signal is shown in orange. Approximately 90% of all molecules mapping to a particular region contain the same orange signal near a mapped nick-label. The Alu repeat signal forms a pattern concordant with predicted Alu sites.

Thus, CRISPR-dCas labeling of DNA in accordance with some embodiments herein can label repeated elements.

### Materials and Methods for Examples 1 and 2

### DNA samples, purification

Ultra-high molecular weight DNA was prepared from BAC clones CH17-353B19 and RP11-963J21 (invitrogen), and human cell line CHM1. Using the Bionano plug lysis protocol and reagents, cells were immobilized in low melting point agarose, lysed, and treated with Proteinase K, before being washed, solubilized with A garase (Thermo Fisher, Wilmington, MA), and drop-dialyzed.

### CRISPR-RNA (crRNA) preparation

The 20nt of crRNA target sequence was designed from website of crispr.mit.edu. DUF crRNA for the genomic sequences of DUF1220 domain (AAGUUCCUUUUAUGCAUUGG, SEQ ID NO: 1), Alu crRNA for the sequence (UGUAAUCCCAGCACUUUGGG, SEQ ID NO: 2), both were synthesized by Synthego (Menlo Park, CA).

### Fluorescent Guide RNA preparation

The universal ATTO^{™}647 labeled tracrRNA (Alt-R CRISPR-Cas9 tracrRNA - ATTO 647) was purchased from Integrated DNA Technologies (Coralville, IA). The fluorescent Alu and DUF gRNAs were created by pre-incubating the tracrRNA-Atto647 (5 pmol) and corresponding crRNA (5 pmol) with 1× NEB Buffer 2 and 1× BSA at 4°C for 30 min.

### Fluorescent CRISPR-dCas9 RNP Assembly

The *S. pyogenes* Cas9 protein containing the double nuclease mutation (D10A and H840A; dCas9) was purchased from PNA Bio Inc (#CD01, Thousand Oaks, CA) and was diluted to a stock concentration of 1 mg/mL or 6 µM with the provided diluent. The fluorescent gRNAs (50 pmol) were mixed with 600 ng of dCas9 (3.7 pmol), 1× NEB Buffer 3 and 1× BSA (NEB) and incubated for 60 min at 37°C. This Fluorescent CRISPR RNPs are stable at 4°C for up to 4 weeks or at -80°C for long-term.

### dCas9fluorescent binding of BAC DNA or human genomic DNA

The 150ng linearized DNA was mixed with 50 ng of the ATTO647-RNP and incubated at 37°C for 60 min. The DNA backbone was stained with YOYO-1, and is shown in blue. The stained samples were loaded and imaged inside the nanochannels.

### The two color genome mapping with dCas9fluorescent binding and sequence-motif labeling

The human genomic DNA sample was nicked with Nt. BspQI (NEB) at 37°C for 2 hours. The nicked DNA was then labeled with Taq DNA Polymerase (NEB), ATTO532 dUTP dAGC and 1× Thermopol Buffer (NEB) for 60 min at 72°C. The nicks were repaired with NAD+, dNTPs and Taq DNA Ligase at 37°C for 30 min. The sample was then treated with Qiagen Protease K for 30 min and inactivate with PMSF for 30 min. The sample was dialyzed in TE on a 0.1 µm NC membrane (Millipore) for 2 hours. After dialysis, 60-100 ng of DNA samples were incubated with 50ng of ATTO647-RNP in 1× NEB Buffer 3 and 1× BSA (NEB) at 37°C for 60 min. The DNA backbone was stained with YOYO-1, and is shown in blue. The DNA backbone staining step was delayed until after CRISPR-binding. The stained samples were loaded and imaged inside the nanochannels.

### RNP binding of DNA and DNA backbone staining

150 ng of DNA (from BACs and human, separately) was combined with 0.4 µL (0.15 pmol) of RNP and incubated at 37°C for 60 min. The DNA backbone was stained using DNA Stain from the Bionano NLRS kit. The sample was stored at room temperature overnight and moved to 4°C for long-term storage.

### Data acquisition and visualization

DUF1220 mapping: 16 µL of the sample was loaded into an IRYS Chip flow cell. An IRYS run was initiated and after 20 cycles of electrophoresis and imaging, molecules were aligned to the reference sequence using IrysView.

Alu repeat mapping: 19 µL of the sample was loaded into a SAPHYR Chip flow cell. A SAPHYR run was initiated through Bionano Access software. After 30 cycles of electrophoresis and imaging, a de novo assembly was performed by Bionano Solve software. Consensus maps and molecules were aligned to the reference sequence and visualized using Bionano Access.

## Claims

1. A method of labeling a DNA at a target sequence, comprising:
contacting a DNA comprising a first target sequence with:
a first dCas protein; and
a first labeled guide RNA (gRNA) comprising a first crRNA and a first tracrRNA comprising a first label, wherein the first crRNA is complementary to the first target sequence or a portion thereof,
incubating the DNA, the first dCas protein, and the first labeled gRNA, whereby the first dCas protein, the DNA, and the first labeled gRNA form a complex wherein the first crRNA is hybridized to the first target sequence or the portion thereof, thereby labeling the DNA at the first target sequence to form a labeled DNA; and
labeling the DNA at another target sequence by nick labeling or direct enzyme labeling.

2. The method of claim 1, wherein the DNA comprises a second target sequence and the method further comprises:
contacting the DNA with:
a second dCas protein; and
a second labeled gRNA comprising a second crRNA and a second tracrRNA comprising a second label that is different from the first label, wherein the second crRNA is complementary to a second target sequence or a portion thereof; and
incubating the DNA with the second dCas protein and the second labeled gRNA, wherein the second dCas protein, the DNA, and the second labeled gRNA form a second complex, wherein the second crRNA is hybridized to the second target sequence or the portion thereof,
thereby labeling the DNA at the second target sequence with the second label, optionally
wherein the DNA is contacted with the first labeled gRNA and the second labeled gRNA at the same time, and wherein the DNA is incubated with the first dCas protein, the second dCas protein, the first labeled gRNA, and the second labeled gRNA at the same time or sequentially.

3. The method of either claim 1 or claim 2:
(i) wherein the DNA remains intact throughout the method;
(ii) wherein at least one of the first and second target sequences is in a region comprising multiple repeats or structural variants not amenable to enzymatic motif labeling;
(iii) wherein labeling the DNA at another target sequence comprises nick labeling and at least one of the first and second target sequences is in a region predicted to form or susceptible of forming a fragile site upon nick translation labeling;
(iv) wherein labeling the DNA at another target sequence comprises nick labeling and at least one of the first and second target sequences is comprised by a genomic region that does not comprise unevenly distributed labels upon nick labeling; and/or
(v) wherein the first target sequence, the second target sequence, or both is in a region comprising multiple repeats or structural variants not amenable to enzymatic motif labeling, optionally wherein the enzymatic motif labeling is nickase labeling.

4. The method of any one of claims 1-3:
(i) wherein at least one of the first and second target sequences is in a region predicted to form a fragile site upon nick translation labeling;
(ii) wherein at least one of the first and second target sequences is comprised by a repeated sequence;
(iii) wherein the labeled DNA is linearized in a fluidic nanochannel, and further optionally wherein the labeled DNA remains intact upon linearization; and/or
(iv) further comprising linearizing the labeled DNA, and detecting a relative distance between the first label and the second label on the linearized DNA in the fluidic nanochannel, optionally wherein the fluidic nanochannel has a length of at least 10 nm and a cross-sectional diameter of less than 1000 nm.

5. The method of any one of claims 1-4, wherein:
(i) the first and/or second dCas protein is dCas9;
(ii) the first and/or second dCas protein comprises one or more mutations and/or one or more deletions in a HNH domain and/or RuvC-like domain; and/or
(iii) the first and/or second dCas protein is not labeled.

6. The method of any one of claims 1-5:
(i) wherein the first and/or the second crRNA comprises a sequence of about 10-40 nucleotides that is complementary to the first and/or the second target sequence;
(ii) wherein said incubating comprises one of more of the DNA, the first dCas protein, the second dCas protein, the first labeled gRNA, and the second labeled gRNA at a concentration of about 5 nM to 500 nM;
(iii) wherein the nick labeling comprises nicking the DNA followed by labeling of the nicks without repairing the labeled DNA, or wherein the nick labeling comprises nicking the DNA followed by labeling of the nicks on the DNA and repairing the labeled DNA; and/or
(iv) wherein the direct enzyme labeling comprises labeling with DLE-1 enzyme.

7. The method of any one of claims 1-6, wherein labeling the DNA at another target sequence comprises nick labeling and the nick labeling comprises:
nicking the DNA with a nickase; and
incubating the nicked DNA with a polymerase and labeled nucleotides, wherein the nucleotides are labeled with a nucleotide label that is the same as or different from the first label and/or the second label, whereby the polymerase incorporates the labeled nucleotides into the DNA in a 5' -> 3' direction.

8. The method of any one of claims 1-7, wherein the first label and/or the second label is selected from the group consisting of: a fluorophore, a quantum dot, a dendrimer, a nanowire, a bead, a hapten, a streptavidin, an avidin, a neutravidin, a biotin, and a reactive group a peptide, a protein, a magnetic bead, a radiolabel, a non-optical label, and a combination thereof.

9. A DNA composition comprising:
a DNA molecule;
a first dCas protein; and
a first labeled guide RNA (gRNA) comprising a first crRNA and a first tracrRNA comprising a first label,
wherein the first dCas protein, the first labeled gRNA, and the first DNA molecule are comprised by a complex comprising the first crRNA hybridized to a first target sequence of the DNA molecule or a portion thereof,
wherein the DNA molecule has been labeled by nick labeling or direct enzyme labeling, optionally by direct enzyme labeling with DLE-1 enzyme.

10. The DNA composition of claim 9, further comprising:
a second dCas protein; and
a second labeled gRNA comprising a second cRNA and a second tracrRNA labeled with a second label different from the first label,
wherein the second dCas protein, the second labeled gRNA, and the DNA molecule are comprised by a complex comprising the second crRNA hybridized to a second target sequence of the DNA molecule or a portion thereof.

11. The DNA composition of either claim 9 or 10, wherein:
(i) the DNA molecule is linearized in a fluidic nanochannel, optionally wherein the DNA molecule is intact in the fluidic nanochannel;
(ii) the fluidic nanochannel has a length of at least 10 nm and a cross-sectional diameter of less than 1000 nm;
(iii) the first and/or second dCas protein is dCas9;
(iv) the first and/or second dCas protein comprises one or more mutations and/or one or more deletions in a HNH domain and/or RuvC-like domain;
(v) the first and/or second dCas protein is not labeled; and/or
(vi) at least one of the first crRNA and the second crRNA comprises a sequence of about 10-40 nucleotides that is complementary to the first and/or the second target sequence or the portion thereof.

12. The DNA composition of any one of claims 9-11, wherein the DNA molecule comprises labeled nucleotides, wherein the nucleotides are labeled with a nucleotide label that is the same as or different from the first label.

13. The DNA composition of any one of claims 9-12, wherein:
the first label and/or the second label is selected from the group consisting of: a fluorophore, a quantum dot, a dendrimer, a nanowire, a bead, a hapten, a streptavidin, an avidin, a neutravidin, a biotin, and a reactive group a peptide, a protein, a magnetic bead, a radiolabel, a non-optical label, and a combination thereof.

14. A kit for performing a method of any one of claims 1-8, the kit comprising:
a first dCas protein, optionally wherein the first dCas protein is dCas9;
a first gRNA comprising a first crRNA and a first tracrRNA;
a first label, optionally wherein the first label is not attached to the first dCas protein and/or the first gRNA comprises the first label, and further optionally wherein the first tracrRNA of the first gRNA comprises the first label; and
DLE-1 enzyme.

15. The kit of claim 14, further comprising:
(i) a fluidic nanochannel;
(ii) nickase;
(iii) a second dCas protein, optionally wherein the second dCas protein is dCas9;
(iv) a second gRNA comprising a second crRNA and a second tracrRNA; and/or
(v) a second label, optionally wherein the second label is comprised by the second gRNA.

## Patentansprüche

1. Verfahren zum Markieren einer DNA an einer Zielsequenz, umfassend:
Inkontaktbringen einer DNA, die eine erste Zielsequenz umfasst, mit:
einem ersten dCas-Protein; und
einer ersten markierten Guide-RNA (gRNA), die eine erste crRNA und eine erste tracrRNA umfasst, die eine erste Markierung umfasst, wobei die erste crRNA komplementär zu der ersten Zielsequenz oder einem Teil davon ist;
Inkubieren der DNA, des ersten dCas-Proteins und der ersten markierten gRNA, wobei das erste dCas-Protein, die DNA und die erste markierte gRNA einen Komplex bilden, wobei die erste crRNA mit der ersten Zielsequenz oder dem Teil davon hybridisiert ist, wodurch die DNA an der ersten Zielsequenz markiert wird, um eine markierte DNA zu bilden; und
Markieren der DNA an einer anderen Zielsequenz durch Nick-Markierung oder direkte Enzymmarkierung.

2. Verfahren nach Anspruch 1, wobei die DNA eine zweite Zielsequenz umfasst und das Verfahren ferner Folgendes umfasst:
Inkontaktbringen der DNA mit:
einem zweiten dCas-Protein; und
einer zweiten markierten gRNA, die eine zweite crRNA und eine zweite tracrRNA umfasst, die eine zweite Markierung umfasst, die von der ersten Markierung verschieden ist, wobei die zweite crRNA komplementär zu einer zweiten Zielsequenz oder einem Teil davon ist; und
Inkubieren der DNA mit dem zweiten dCas-Protein und der zweiten markierten gRNA, wobei das zweite dCas-Protein, die DNA und die zweite markierte gRNA einen zweiten Komplex bilden, wobei die zweite crRNA mit der zweiten Zielsequenz oder dem Teil davon hybridisiert ist,
wodurch die DNA an der zweiten Zielsequenz mit der zweiten Markierung markiert wird, optional
wobei die DNA gleichzeitig mit der ersten markierten gRNA und der zweiten markierten gRNA in Kontakt gebracht wird, und wobei die DNA gleichzeitig oder nacheinander mit dem ersten dCas-Protein, dem zweiten dCas-Protein, der ersten markierten gRNA und der zweiten markierten gRNA inkubiert wird.

3. Verfahren nach Anspruch 1 oder Anspruch 2:
i) wobei die DNA bleibt während des gesamten Verfahrens intakt bleibt;
(ii) wobei sich mindestens eine von der ersten und der zweiten Zielsequenz in einer Region befindet, die mehrere Wiederholungen oder Strukturvarianten umfasst, die sich nicht für eine enzymatische Motivmarkierung eignen;
(iii) wobei Markieren der DNA an einer anderen Zielsequenz eine Nick-Markierung umfasst und sich mindestens eine von der ersten und der zweiten Zielsequenz in einer Region befindet, von der vorhergesagt wird, dass sie bei einer Nick-Translation-Markierung eine fragile Stelle bildet oder die anfällig dafür ist, eine solche zu bilden;
(iv) wobei Markieren der DNA an einer anderen Zielsequenz eine Nick-Markierung umfasst und mindestens eine von der ersten und der zweiten Zielsequenz von einer genomischen Region umfasst wird, die bei einer Nick-Markierung keine ungleichmäßig verteilten Markierungen umfasst; und/oder
(v) wobei die erste Zielsequenz, die zweite Zielsequenz oder beide in einer Region liegen, die mehrere Wiederholungen oder Strukturvarianten umfasst, die nicht für eine enzymatische Motivmarkierung geeignet sind, wobei die enzymatische Motivmarkierung optional eine Nickasemarkierung ist.

4. Verfahren nach einem der Ansprüche 1-3:
(i) wobei sich mindestens eine von der ersten und der zweiten Zielsequenz in einer Region befindet, von der vorhergesagt wird, dass sie bei einer Nick-Translation-Markierung eine fragile Stelle bildet;
(ii) wobei mindestens eine von der ersten und der zweiten Zielsequenz von einer wiederholten Sequenz umfasst wird;
(iii) wobei die markierte DNA in einem fluidischen Nanokanal linearisiert wird, und wobei die markierte DNA optional nach der Linearisierung intakt bleibt; und/oder
(iv) ferner umfassend Linearisieren der markierten DNA und Erfassen eines relativen Abstands zwischen der ersten Markierung und der zweiten Markierung auf der linearisierten DNA in dem fluidischen Nanokanal, wobei der fluidische Nanokanal optional eine Länge von mindestens 10 nm und einen Querschnittsdurchmesser von weniger als 1000 nm aufweist.

5. Verfahren nach einem der Ansprüche 1-4, wobei:
(i) das erste und/oder das zweite dCas-Protein dCas9 sind/ist;
(ii) das erste und/oder das zweite dCas-Protein eine oder mehrere Mutationen und/oder eine oder mehrere Deletionen in einer HNH-Domäne und/oder einer RuvC-ähnlichen Domäne umfassen/umfasst; und/oder
(iii) das erste und/oder das zweite dCas-Protein nicht markiert sind/ist.

6. Verfahren nach einem der Ansprüche 1-5:
(i) wobei die erste und/oder die zweite crRNA eine Sequenz von etwa 10-40 Nukleotiden umfassen/umfasst, die komplementär zu der ersten und/oder der zweiten Zielsequenz ist;
(ii) wobei das Inkubieren eines oder mehrere von der DNA, dem ersten dCas-Protein, dem zweiten dCas-Protein, der ersten markierten gRNA und der zweiten markierten gRNA in einer Konzentration von etwa 5 nM bis 500 nM umfasst;
(iii) wobei das Nick-Markieren Nicken der DNA, gefolgt vom Markieren der Nicks ohne Reparieren der markierten DNA umfasst, oder wobei das Nick-Markieren Nicken der DNA, gefolgt vom Markieren der Nicks auf der DNA und Reparieren der markierten DNA umfasst; und/oder
(iv) wobei die direkte Enzymmarkierung Markieren mit dem DLE-1-Enzym umfasst.

7. Verfahren nach einem der Ansprüche 1-6, wobei Markieren der DNA an einer anderen Zielsequenz Nick-Markieren umfasst und das Nick-Markieren Folgendes umfasst:
Nicken der DNA mit einer Nickase; und
Inkubieren der genickten DNA mit einer Polymerase und markierten Nukleotiden, wobei die Nukleotide mit einer Nukleotidmarkierung markiert sind, die gleich oder verschieden von der ersten Markierung und/oder der zweiten Markierung ist, wobei die Polymerase die markierten Nukleotide in einer 5'->3'-Richtung in die DNA einbaut.

8. Verfahren nach einem der Ansprüche 1-7, wobei die erste Markierung und/oder die zweite Markierung aus der Gruppe ausgewählt sind/ist, die aus Folgendem besteht: einem Fluorophor, einem Quantenpunkt, einem Dendrimer, einem Nanodraht, einer Perle, einem Hapten, einem Streptavidin, einem Avidin, einem Neutravidin, einem Biotin und einer reaktiven Gruppe, einem Peptid, einem Protein, einer magnetischen Perle, einer radioaktiven Markierung, einer nicht-optischen Markierung und einer Kombination davon.

9. DNA-Zusammensetzung, umfassend:
ein DNA-Molekül;
ein erstes dCas-Protein; und
eine erste markierte Guide-RNA (gRNA), die eine erste crRNA und eine erste tracrRNA umfasst, die eine erste Markierung umfasst,
wobei das erste dCas-Protein, die erste markierte gRNA und das erste DNA-Molekül von einem Komplex umfasst sind, der die erste crRNA umfasst, die an eine erste Zielsequenz des DNA-Moleküls oder einen Teil davon hybridisiert ist,
wobei das DNA-Molekül durch Nick-Markierung oder direkte Enzymmarkierung markiert wurde, optional durch direkte Enzymmarkierung mit dem DLE-1-Enzym.

10. DNA-Zusammensetzung nach Anspruch 9, ferner umfassend:
ein zweites dCas-Protein; und
eine zweite markierte gRNA, die eine zweite cRNA und eine zweite tracrRNA umfasst, die mit einer zweiten Markierung markiert ist, die von der ersten Markierung verschieden ist,
wobei das zweite dCas-Protein, die zweite markierte gRNA und das DNA-Molekül von einem Komplex umfasst sind, der die zweite crRNA umfasst, die mit einer zweiten Zielsequenz des DNA-Moleküls oder einem Teil davon hybridisiert ist.

11. DNA-Zusammensetzung nach Anspruch 9 oder 10, wobei:
(i) das DNA-Molekül in einem fluidischen Nanokanal linearisiert wird, wobei das DNA-Molekül in dem fluidischen Nanokanal optional intakt ist;
(ii) der fluidische Nanokanal eine Länge von mindestens 10 nm und einen Querschnittsdurchmesser von weniger als 1000 nm aufweist;
(iii) das erste und/oder das zweite dCas-Protein dCas9 sind/ist;
(iv) das erste und/oder das zweite dCas-Protein eine oder mehrere Mutationen und/oder eine oder mehrere Deletionen in einer HNH-Domäne und/oder RuvC-ähnlichen Domäne umfassen/umfasst;
(v) das erste und/oder das zweite dCas-Protein nicht markiert sind/ist; und/oder
(vi) mindestens eine der ersten crRNA und der zweiten crRNA eine Sequenz von etwa 10-40 Nukleotiden umfasst, die komplementär zu der ersten und/oder der zweiten Zielsequenz oder dem Teil davon ist.

12. DNA-Zusammensetzung nach einem der Ansprüche 9-11, wobei das DNA-Molekül markierte Nukleotide umfasst, wobei die Nukleotide mit einer Nukleotidmarkierung markiert sind, die mit der ersten Markierung identisch oder von dieser verschieden ist.

13. DNA-Zusammensetzung nach einem der Ansprüche 9-12, wobei:
die erste Markierung und/oder die zweite Markierung aus der Gruppe ausgewählt sind/ist, die aus Folgendem besteht: einem Fluorophor, einem Quantenpunkt, einem Dendrimer, einem Nanodraht, einer Perle, einem Hapten, einem Streptavidin, einem Avidin, einem Neutravidin, einem Biotin und einer reaktiven Gruppe, einem Peptid, einem Protein, einer magnetischen Perle, einer radioaktiven Markierung, einer nicht-optischen Markierung und einer Kombination davon.

14. Kit zum Durchführen eines Verfahrens nach einem der Ansprüche 1-8, wobei das Kit Folgendes umfasst:
ein erstes dCas-Protein, wobei das erste dCas-Protein optional dCas9 ist;
eine erste gRNA, die eine erste crRNA und eine erste tracrRNA umfasst;
eine erste Markierung, wobei die erste Markierung optional nicht an dem ersten dCas-Protein angebracht ist, und/oder die erste gRNA die erste Markierung umfasst, und wobei ferner optional die erste tracrRNA der ersten gRNA die erste Markierung umfasst; und
DLE-1-Enzym.

15. Kit nach Anspruch 14, ferner umfassend:
(i) einen fluidischen Nanokanal;
(ii) Nickase;
(iii) ein zweites dCas-Protein, wobei das zweite dCas-Protein optional dCas9 ist;
(iv) eine zweite gRNA, die eine zweite crRNA und eine zweite tracrRNA umfasst; und/oder
(v) eine zweite Markierung, wobei die zweite Markierung optional von der zweiten gRNA umfasst ist.

## Revendications

1. Procédé de marquage d'un ADN au niveau d'une séquence cible, comprenant :
la mise en contact d'un ADN comprenant une première séquence cible avec :
une première protéine dCas ; et
un premier ARN guide (ARNg) marqué comprenant un premier ARNcr et un premier ARNtracr comprenant un premier marqueur, ledit premier ARNcr étant complémentaire à la première séquence cible ou à une partie de celle-ci ;
l'incubation de l'ADN, de la première protéine dCas et du premier ARNg marqué, grâce à laquelle la première protéine dCas, l'ADN et le premier ARNg marqué forment un complexe dans lequel le premier ARNcr est hybridé à la première séquence cible ou à la partie de celle-ci, marquant ainsi l'ADN au niveau de la première séquence cible pour former un ADN marqué ; et
le marquage de l'ADN au niveau d'une autre séquence cible par marquage par coupure ou marquage enzymatique direct.

2. Procédé selon la revendication 1, ledit ADN comprenant une seconde séquence cible et le procédé comprenant en outre :
la mise en contact de l'ADN avec :
une seconde protéine dCas ;
un second ARNg marqué comprenant un second ARNcr et un second ARNtracr comprenant un second marqueur qui est différent du premier marqueur, ledit second ARNcr étant complémentaire à une seconde séquence cible ou à une partie de celle-ci ; et
l'incubation de l'ADN avec la seconde protéine dCas et le second ARNg marqué, ladite seconde protéine dCas, ledit ADN et ledit second ARNg marqué formant un second complexe dans lequel le second ARNcr est hybridé à la seconde séquence cible ou à la partie de celle-ci,
ainsi le marquage de l'ADN au niveau de la seconde séquence cible avec le second marqueur, éventuellement
ledit ADN étant mis en contact avec le premier ARNg marqué et le second ARNg marqué en même temps, et ledit ADN étant incubé avec la première protéine dCas, la seconde protéine dCas, le premier ARNg marqué et le second ARNg marqué en même temps ou de manière séquentielle.

3. Procédé selon la revendication 1 ou la revendication 2 :
(i) ledit ADN restant intact tout au long du procédé ;
(ii) au moins l'une desdites première et seconde séquences cibles se trouvant dans une région comprenant de multiples répétitions ou variants structurels ne se prêtant pas à un marquage de motif enzymatique ;
(iii) ledit marquage de l'ADN au niveau d'une autre séquence cible comprenant le marquage par coupure et au moins l'une desdites première et seconde séquences se trouvant dans une région prédite pour former ou susceptible de former un site fragile lors du marquage de traduction par coupure ;
(iv) ledit marquage de l'ADN au niveau d'une autre séquence cible comprenant le marquage par coupure et au moins l'une desdites première et seconde séquences cibles étant constituée d'une région génomique qui ne comprend pas de marqueurs répartis inégalement lors du marquage par coupure ; et/ou
(v) ladite première séquence cible, ladite seconde séquence cible ou les deux se trouvant dans une région comprenant de multiples répétitions ou variants structurels ne se prêtant pas à un marquage de motif enzymatique, éventuellement ledit marquage de motif enzymatique étant un marquage par nickase.

4. Procédé selon l'une quelconque des revendications 1 à 3 :
(i) au moins l'une desdites première et seconde séquences cibles se trouvant dans une région prédite pour former un site fragile lors du marquage de traduction par coupure ;
(ii) au moins l'une desdites première et seconde séquences cibles étant constitué d'une séquence répétée ;
(iii) ledit ADN marqué étant linéarisé dans un premier nanocanal fluidique, et en outre éventuellement ledit ADN marqué restant intact lors de la linéarisation ; et/ou
(vi) comprenant en outre la linéarisation de l'ADN marqué, et la détection de la distance relative entre le premier marqueur et le second marqueur sur l'ADN linéarisé dans le nanocanal fluidique, éventuellement ledit nanocanal fluidique comportant une longueur d'au moins 10 nm et un diamètre de section transversale inférieur à 1000 nm.

5. Procédé selon l'une quelconque des revendications 1 à 4 :
(i) lesdites première et/ou seconde protéines dCas étant la dCas9 ;
(ii) lesdites première et/ou seconde protéines dCas comprenant une ou plusieurs mutations et/ou une ou plusieurs délétions dans un domaine HNH et/ou un domaine de type RuvC ; et/ou
(iii) lesdites première et/ou seconde protéines dCas n'étant pas marquées.

6. Procédé selon l'une quelconque des revendications 1 à 5 :
(i) ledit premier et/ou ledit second ARNcr comprenant une séquence d'environ 10 à 40 nucléotides qui est complémentaire à la première et/ou à la seconde séquence cible ;
(ii) ladite incubation comprenant un ou plusieurs éléments parmi l'ADN, la première protéine dCas, la seconde protéine dCas, le premier ARNg marqué et le second ARNg marqué à une concentration d'environ 5 nM à 500 nM ;
(iii) ledit marquage par coupure comprenant la coupure de l'ADN suivie par le marquage des coupures sans réparer l'ADN marqué, ou ledit marquage par coupure comprenant la coupure de l'ADN suivie du marquage des coupures sur l'ADN et la réparation de l'ADN marqué ; et/ou
(iv) ledit marquage enzymatique direct comprenant le marquage avec l'enzyme DLE-1.

7. Procédé selon l'une quelconque des revendications 1 à 6, ledit marquage de l'ADN au niveau d'une autre séquence cible comprenant le marquage par coupure et ledit marquage par coupure comprenant :
la coupure de l'ADN avec une nickase ; et
l'incubation de l'ADN coupé avec une polymérase et des nucléotides marqués, lesdits nucléotides étant marqués avec un marqueur de nucléotide qui est identique au premier marqueur et/ou au second marqueur ou différent du premier marqueur et/ou du second marqueur, ladite polymérase incorporant les nucléotides marqués dans l'ADN dans une direction 5' -> 3'.

8. Procédé selon l'une quelconque des revendications 1 à 7, ledit premier marqueur et/ou ledit second marqueur étant choisi dans le groupe constitué par : un fluorophore, un point quantique, un dendrimère, un nanofil, une bille, un haptène, une streptavidine, une avidine, une neutravidine, une biotine et un groupe réactif, un peptide, une protéine, une bille magnétique, un radiomarqueur, un marqueur non optique et une combinaison de ceux-ci.

9. Composition d'ADN comprenant :
une molécule d'ADN ;
une première protéine dCas ; et
un premier ARN guide (ARNg) marqué comprenant un premier ARNcr et un premier ARNtracr comprenant un premier marqueur,
ladite première protéine dCas, ledit premier ARNg marqué et ladite première molécule d'ADN étant constituée d'un complexe comprenant le premier ARNcr hybridé à une première séquence cible de la molécule d'ADN ou d'une partie de celle-ci,
ladite molécule d'ADN ayant été marquée par marquage par coupure ou par marquage enzymatique direct, éventuellement par marquage enzymatique direct avec l'enzyme DLE-1.

10. Composition d'ADN selon la revendication 9, comprenant en outre :
une seconde protéine dCas ; et
un second ARNg marqué comprenant un second ARNcr et un second ARNtracr marqué avec un second marqueur différent du premier marqueur,
ladite seconde protéine dCas, ledit second ARNg marqué et ladite molécule d'ADN étant constituée d'un complexe comprenant le second ARNcr hybridé à une seconde séquence cible de la molécule d'ADN ou d'une partie de celle-ci.

11. Composition d'ADN selon la revendication 9 ou 10 :
(i) ladite molécule d'ADN étant linéarisée dans un nanocanal fluidique, éventuellement ladite molécule d'ADN étant intacte dans le nanocanal fluidique ;
(ii) ledit nanocanal fluidique comportant une longueur d'au moins 10 nm et un diamètre de section transversal inférieur à 1000 nm ;
(iii) lesdites première et/ou seconde protéines dCas étant la dCas9 ;
(iv) lesdites première et/ou seconde protéines dCas comprenant une ou plusieurs mutations et/ou une ou plusieurs délétions dans un domaine HNH et/ou un domaine de type RuvC ;
(v) lesdites première et/ou seconde protéines dCas n'étant pas marquées ; et/ou
(vi) au moins l'un parmi le premier ARNcr et le second ARNcr comprenant une séquence d'environ 10 à 40 nucléotides qui est complémentaire à la première et/ou à la seconde séquence cible ou à la partie de celle-ci.

12. Composition d'ADN selon l'une quelconque des revendications 9 à 11, ladite molécule d'ADN comprenant des nucléotides marqués, lesdits nucléotides étant marqués avec un marqueur de nucléotide qui est identique au premier marqueur ou différent de celui-ci.

13. Composition d'ADN selon l'une quelconque des revendications 9 à 12 :
ledit premier marqueur et/ou ledit second marqueur étant choisis dans le groupe constitué par : un fluorophore, un point quantique, un dendrimère, un nanofil, une bille, un haptène, une streptavidine, une avidine, une neutravidine, une biotine et un groupe réactif, un peptide, une protéine, une bille magnétique, un radiomarqueur, un marqueur non optique et une combinaison de ceux-ci.

14. Kit permettant d'effectuer un procédé selon l'une quelconque des revendications 1 à 8, le kit comprenant :
une première protéine dCas, éventuellement ladite première protéine dCas étant la Dcas9 ;
un premier ARNg comprenant un premier ARNcr et un premier ARNtracr ;
un premier marqueur, éventuellement ledit premier marqueur n'étant pas lié à la première protéine dCas et/ou ledit premier ARNg comprenant le premier marqueur, et éventuellement en outre ledit premier ARNtracr du premier ARNg comprenant le premier marqueur ; et
l'enzyme DLE-1.

15. Kit selon la revendication 14, comprenant en outre :
(i) un nanocanal fluidique ;
(ii) une nickase ;
(iii) une seconde protéine dCas, éventuellement ladite seconde protéine dCas étant la dCas9 ;
(iv) un second ARNg comprenant un second ARNcr et un second ARNtracr ; et/ou
(v) un second marqueur, éventuellement ledit second marqueur étant constitué du second ARNg.
